(19) Europäisches Patentamt
European Patent Office
Office européen des brevets

(11) Publication number: **0 143 615**
**A2**

(12) **EUROPEAN PATENT APPLICATION**

(21) Application number: **84308110.0**

(22) Date of filing: **22.11.84**

(51) Int. Cl.⁴: **C 12 N 15/00**, C 12 N 1/20, C 12 Q 1/68 // (C12N1/20, C12R1:41)

(30) Priority: **23.11.83 US 554984**
**23.10.84 US 662611**

(43) Date of publication of application: **05.06.85**
**Bulletin 85/23**

(84) Designated Contracting States: **AT BE CH DE FR GB IT LI LU NL SE**



(71) Applicant: **Agrigenetics Research Associates Limited, 3375 Mitchell Lane, Boulder Colorado (US)**

(72) Inventor: **Shine, John, 406 Deodara Drive, Los Altos California 94022 (US)**
Inventor: **Schofield, Peter, 6 Whittle Street, Hughes A.C.T. 2605 (AU)**
Inventor: **Watson, John, 12 Foxall Street, Holder A.C.T. 2611 (AU)**

(74) Representative: **De Minvielle-Devaux, Ian Benedict Peter et al, CARPMAELS & RANSFORD 43, Bloomsbury Square, London WC1A 2RA (GB)**


(54) **Repetitive sequences derived from certain nitrogen fixing bacterial species.**


(57) The invention provides a bacterial strain.

A bacterial strain containing and replicating therein a recombinant DNA plasmid comprising:

(a) a vector and
(b) a RDS derived from a symbiotic Sym plasmid recovered from a nitrogen fixing bacterial species.

Nod⁻
Nod⁺
ANU 851 Tn5
ANU882 Km
RS1
RS3
Fix
pR1587
NODULATION
pR1572
pR1585
pR1270
pR1514
0 2kb
pR1608
pR1011
pR1578
pR1564
pR1580


EP 0 143 615 A2

# REPETITIVE SEQUENCES OF

# RHIZOBIUM TRIFOLII

## Field of the Invention

Nitrogen fixing bacteria are able to reduce dinitrogen to ammonia. In some cases, e.g., Klebsiella pneumoniae, free-living bacteria can carry out this reduction, but in other cases, e.g., Rhizobium trifolii, the reduction occurs when the bacteria are in association with plant roots. Many of these bacterial-plant symbioses are between Rhizobium species and legumes and the association is specific. A particular legume species may be nodulated by some Rhizobium isolates but not by others. For example, some rhizobial isolates can infect and nodulate soybeans but cannot nodulate garden peas or white clover. The basis of this specificity may involve recognition and binding of the bacterial cell by some component, possibly a lectin, in the plant roots. Thus, fluorescein-labelled soybean agglutinin binds to 22/25 strains of Rhizobium japonicum which infect soybeans but does not bind to any of the 23 strains from 5 species of Rhizobium that infect other legumes (Bohlool, B. B. and Schmidt, E. L. (1974) Science 185:269-271).

In addition to recognition specificity, there appear to be a variety of specific host-symbiont interactions which occur during nodulation. For example, rhizobial responses effected by the host plant include morphological changes during conversion to the bacteroid state, and the induction of nitrogenase. Plant responses affected by interaction with Rhizobium include nodule development and synthesis of leghemoglobin. Studies of the temporal unfolding of these responses during nodulation suggest that each stage of the process is mediated by a complex series of feedback signals from the host plant to the bacterial symbiont, and from the bacteria to the plant. Many of these signals appear to be specific for each host-symbiont species pair and to account for much of the observed host species specificity of most Rhizobium strains.

In general, identification of a bacterial isolate as Rhizobium can only be achieved by demonstration of nodule formation and re-isolation of the same bacterium from the nodules. Furthermore, due to the specificity of the bac- rium-host plant interaction, a number of different legume species must be tested. There are a number of characteristics, however, that clearly indic that a bacterial isolate is a non-Rhizobium species (Vincent, J. M., Nutman P. S. and F. A. Skinner (1979) in "Identification methods for Microbiologists", F. A. Skinner and D. W. Lovelock, eds. Academic Press, London).

Rhizobia are short to medium gram-negative rods so contra-indications include gram positiveness, endospores, large rods or cocci and chain formation. A rapid growth rate in one or two days and the production of col are also indications that the isolate is not a Rhizobium species.

## Background of the Invention

Rhizobial isolates can easily be obtained from freshly-collected turgid nodules of a healthy plant. Rhizobium species can also be isolated from soi but in this case, other bacteria are likely to swamp out the Rhizobium on growth media. If Rhizobium species are to be isolated from soil, then it is generally desirable to use a legume species as a "trap" host. Again, due to the specificity of the bacterium-host interaction, a number of different legume species must be used or many rhizobial isolates will be lost. It has been stated by an authority on the subject of symbiotic bacteria-plant relationships (J. M. Vincent (1982) in "Nitrogen fixation in legumes" Academic Press, New York, pp. 5-11) that "except under special circumstances (such as when working with an identifiable ("labelled") strain the ability to nodulate a legume remains the final arbiter as to a culture's allocation to the genus Rhizobium and, in some cases, species." A number of methods which are suitable for testing nodulation of large and small seeded species have been described (Vincent, J. M. (1970) "A manual for the practical study of root-nodul bacteria" IBP Handbook No. 15, Blackwell Scientific Publications, Oxford). However, if these tests are not meticulously done, the results are unreliable. For example, a slow-growing and a fast-growing species may occur as a mixture and it is quite easy for the cells of the slow-growing form to remain unobserved within a large, gummy colony. In any event, the tests disclosed in the prior art are time-consuming and tedious.

Rhizobium trifolii strains specifically infect and nodulate clover plants. These strains contain a number of large plasmids ranging in size from about 180 kilobases (kb) to greater than 500kb. The plasmids can be separated and it has been shown that nitrogen fixation (nif) genes are located on a particular plasmid. This plasmid is therefore referred to as the Sym (symbiotic) plasmid (Hooykaas, P. J. J., van Brussel, A. A. N., den Dulk-Ras, H., van Slogteren, G. M. S. and R. A. Schilperoort (1981) Nature 291:351). It is apparent that no quick and reliable method exists which allows identification of a specific symbiotic plasmid occurring in a Rhizobium species with a limited host range.

## Summary of the Invention

Large numbers of bacterial isolates may be recovered from plants or from soil samples. Previously, only expensive and tedious tests were available to definitively identify a specific nitrogen fixing bacterial species capable of a symbiotic interaction with a specific plant species or variety. This invention describes a novel composition and an inexpensive, rapid method for using said composition to screen bacterial isolates having the ability to nodulate one or more legume species.

## Detailed Description of the Invention

The present invention is exemplified by the construction of a recombinant plasmid comprising a vector and a fragment of the symbiotic plasmid of Rhizobium trifolii. The invention is based on the unexpected discovery of a DNA segment, herein designated RDS (Rhizobium diagnostic segment).

In Rhizobium trifolii RDS is found on the symbiotic (Sym) plasmid and is located at more than one position on that plasmid. RDS of a given Rhizobial species is a member of a family of substantially homologous (strongly conserved) nucleotide sequences. That is to say, slight sequence variations are observed between RDS sequences located at different sites on the same Sym plasmid. Substantially homologous nucleotide sequences are defined in term of the conditions which permit hybridization of denatured DNA fragments as described in Example 8. Different RDS which have been obtained from a single strain or different strains of the same species are indeed substantially homologous as measured under the conditions which permit hybridization of denatured DNA fragments as described in Example 8. Examples of such substantial sequence homology of several RDS from the symbiotic (Sym) plasmi of a single strain of Rhizobium trifolii are disclosed herein for portions o three such RDS (see Example 8).

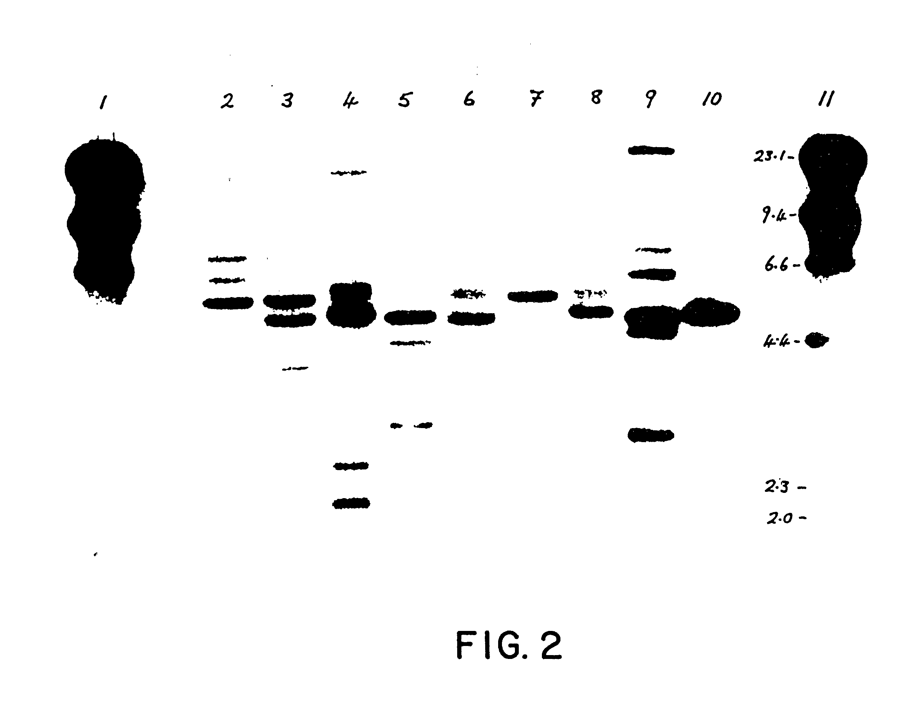

These three RDS are designated RS-1(b), RS-2 and RS-3 (see below). In addition, a single RDS from a second strain of Rhizobium trifolii has been identified and sequenced. This RDS from the said second strain is designate RS-1(a) (see below) and its sequence was unexpectedly found to be included i the nucleotide sequence disclosed in U.S. Patent Application Serial No. 506,676, filed June 22, 1983, Fig. 2. The location, size and utility of RS-1(a) were not known when Serial No. 506,676 was filed.

RDS's isolated from Rhizobial strains of the same species (e.g., R. trifolii) are substantially homologous with one another, but not with RDS's isolated from Rhizobial strains of another species (e.g., R. meliloti). For example, a unique family of RDS (see previous paragraph) is present in Rhizobium strains capable of nodulating one or more species of clover. A plasmid comprising an RDS of R. trifolii can be used as a probe to identify unknown Rhizobial isolates as clover-nodulating Rhizobium regardless of whether that strain was recovered from soil samples or from nodules of leguminous or non-leguminous plants. Similarly, RDS of any other Rhizobium species can be used to identify other members of that species. The isolation characterization and method of using an RDS is described herein.

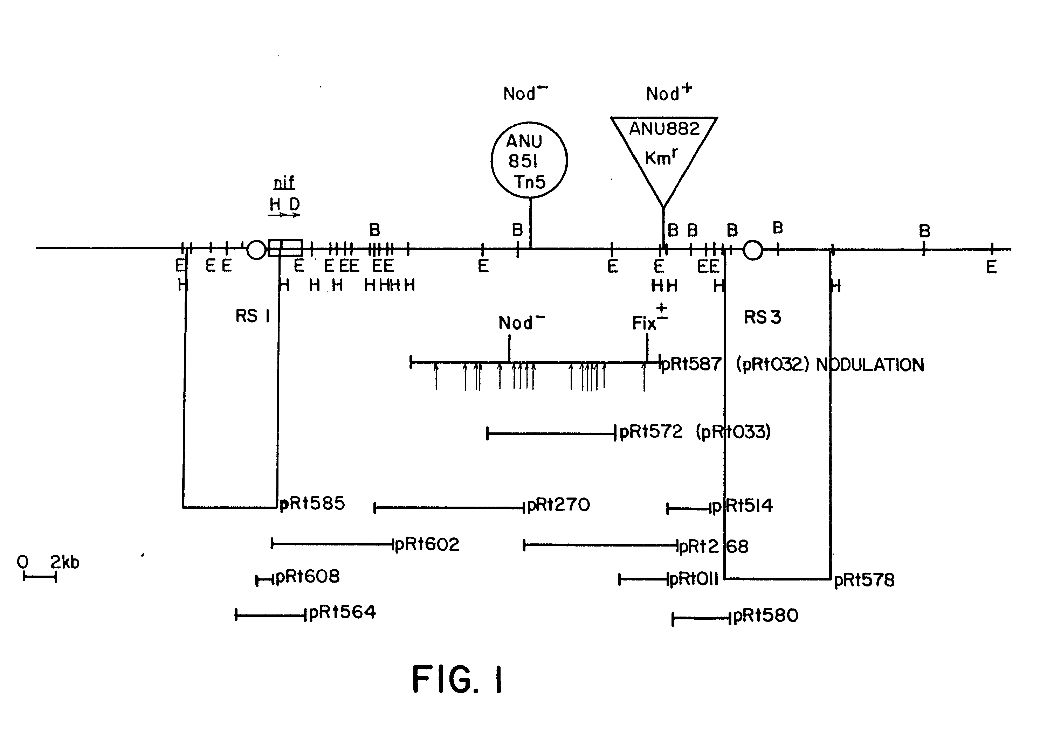

The isolation and recognition of the RDS of R. trifolii was achieved by isolation of the nif genes and the flanking sequences from the Sym plasmid of Rhizobium trifolii. Firstly, transposon-induced mutagenesis produced a variety of nif mutants and restriction fragments of the mutants carrying the transposon were cloned into various vectors and transformed into E. coli where they were amplified. Secondly, wild-type R. trifolii DNA was cleaved with restriction endonucleases and the cloned mutant nif gene fragments were radioactively labelled and used as probes to detect the wild-type nif fragments. Thirdly, these various wild-type nif fragments were radioactively labelled and used as probes to detect complementary sequences which were present in other regions of the Sym plasmid. Unexpectedly, it was discovered that multiple bands containing homologous sequences were detectable when certain nif fragments were used as probes. Mapping experiments showed the presence, on the 5' side of the nifH gene, of a DNA sequence which exists in at least 5-8 locations on the individual Sym plasmids of seven independent isolates of R. trifolii, but not on the Sym plasmids of non-clover-nodulating Rhizobia (Fig. 1).

In strain ANU843, the initially-isolated copy of the RDS (RS-1(b) in Fig. 1) was localized to a 1kb region close to the 5' end of nifH. At least five copies of the RDS were found exclusively on the Sym plasmid of ANU843. No such sequence was detected in total DNA isolated from ANU845, a Sym plasmid-cured derivative of ANU843. A second copy of the RDS (RS-3 in Fig. 1) has been mapped approximately 28kb distant from nifH. The uniqueness of this repeated sequence was shown by the fact that there was no detectable hybridization of this repeated sequence to the DNA of other Rhizobium species, e.g., Rhizobium meliloti or Rhizobium leguminosarum. These two copies of the RDS, which have been mapped, flank a region which carries all of the identified nodulation and nitrogen fixation genes. This observation together with the fact that all copies of the RDS of Rhizobium trifolii are located on the Sym plasmid suggest that such sequences may play a role in the specificity of host-symbiont interaction during nodulation. Since an RDS is unique to Rhizobium isolates that infect and nodulate a specific legume family, it can be used to quickly and efficiently identify other similar bacterial strains regardless of their origin. In principle, a recombinant plasmid comprising RDS (RDS plasmid, hereinafter) is constructed and amplified by replication in a host bacterial cell. Any host cell strain in which the RDS plasmid replicates is suitable for maintaining the plasmid and for generating adequate quantities of plasmid DNA. The plasmid itself may be derived from any stably-replicating plasmid vector, capable of multicopy replication and bearing a genetic marker, such as a drug resistance gene, to permit selection of host cell lines carrying the plasmid. Suitable host cell strains include, but are not limited to, strains of E. coli, Agrobacterium, Rhizobium, and the like. Suitable plasmid vectors include, but are not limited to, narrow host range vectors such as pBR322, pACYC177, etc., and broad host range vectors such as pRK290, RP4, etc. The choice of plasmid vector and host will be matters of choice depending on considerations of operating convenience known and recognized by those of ordinary skill in the art.

An RDS plasmid can be radiolabeled by known means. Entire RDS plasmid DNA or a subfragment thereof comprising RDS, can be used as a hybridization probe to detect the presence or absence of DNA homologous to the RDS, in DN/ extracted from a given Rhizobium strain. The presence of DNA homologous to RDS indicates that the strain from which it was obtained will be capable of nodulating legumes of the family for which that RDS is diagnostic.

The hybridizations may be carried out on unfractionated DNA samples (dc blot hybridization), or on electrophoretically fractionated DNA ("Southern" hybridization). The former is advantageous for screening large numbers of strains, while the latter is advantageous for locating specific fragments th contain RDS-homologous sequences. The most unequivocal identification of a given legume-nodulating strain will be obtained where hybridization is carri out under stringent hybridization conditions, as will be understood by those of ordinary skill in the art. The RDS is sufficiently long that stable hybrids will be formed even where flanking sequences are non-homologous. It will be understood that the most sensitive and specific hybridization will take place where the probe is the RDS itself, or a subfragment of the RDS plasmid containing the RDS.

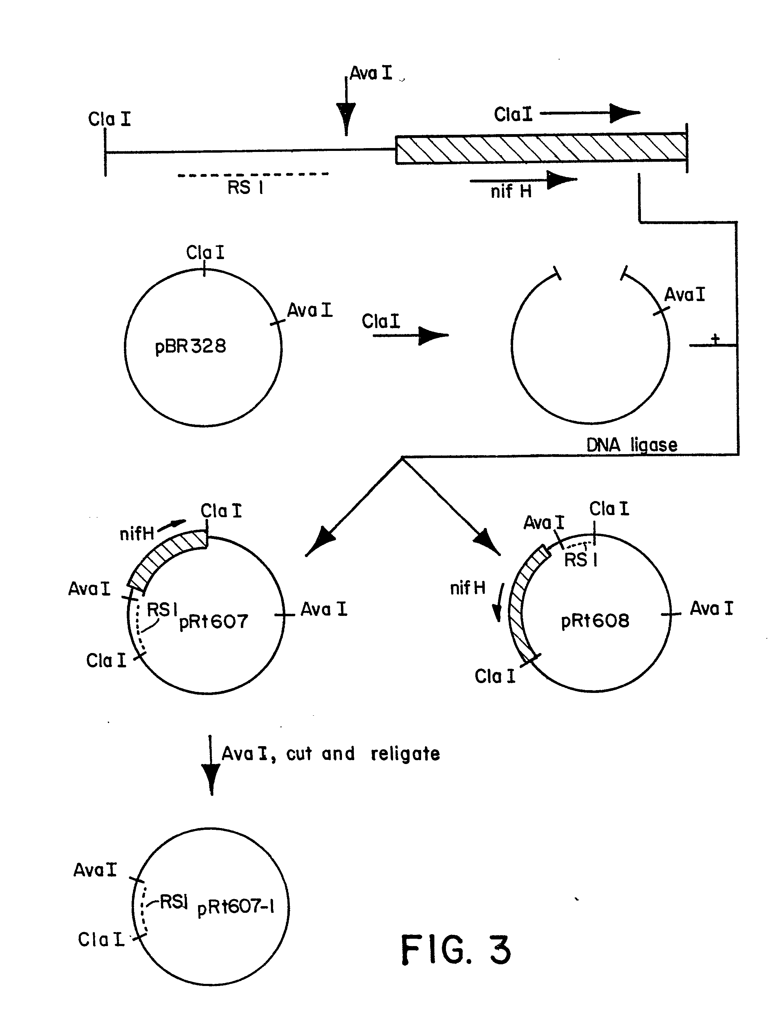

By using the radioactively labelled probe pRt607 (see Example 4 and Fig. 3), it was possible to identify and isolate four of the RDS DNA fragments fr two strains of Rhizobium trifolii. Two of these fragments (RS-1(b) and RS-3) have been mapped at opposite ends of the region which carries all of the identified nodulation and nitrogen fixation genes while the position of the other fragment RS-2 has not been determined. Part of RS-1(a) has been mappec and sequenced at the 5'-end of the nifH gene of a second strain of Rhizobium meliloti (U.S. Patent Application Serial No. 506,676; filed June 22, 1983--incorporated here by reference). However, the existence of RS-1(a) as part c a family of repeated sequences was not known or understood at the time that application was filed. All four of these RDS fragments were partially sequenced and the results are given as follows (Table 1):

```
RS-1(a) 5'-T C T T C A G G A - G C G A C A - G - - A T - - G T G A C C A G - - T T
RS-1(b) 5'-T C T T C A G G A - G C A A C A T G A C A T - - G T G T C C G A C A T T
RS-2    5'-T C T T C A G G A C T A C T C A C G A C A T - - G T G T G C T A C A T T
RS-3    5'-T C T T C A G G G - G C A - C A T G A C A T A T G T G - - C G A C A T T

RS-1(a)    G T C G T C A C C T T T G T C G G C T T C G T G - A C A C G C T - T T A
RS-1(b)    G T C G T T T C C T T T G T C G A C T T C G A G - A C A C G - T - T T A
RS-2       G T C G - - - C C T T T G T C G A C T T C G T G G A C A C G - T C T T A
RS-3       G T C G T [....................unsequenced..................]

RS-1(a)    G G A T T C T T C G G T C C G G T A T T T T A T C C C T C T A A G T G T
RS-1(b)    G G A T T C T T C G G T C C G A T A T T T T A T C T T T C T A A G T G T
RS-2       G G A T T C T T C G G T C C G G T A T T T T A T C C C T C T A A - T G T

RS-1(a)    C T G C G G C A G C A C C A A C T T C C G T T C T G C C C C T T C A A T
RS-1(b)    T T G C G G C A C A - C C A A A T T C C G T T C T G C C A C A T C A A T
RS-2       T T - C G G C A G G - C - A A A T T C C G T T C T G C C A C A T C A A T

RS-1(a)    C A G C T C A A T - T G G C A C C A C G C T T G A A A A T T G T T C T C
RS-1(b)    C C G C C C A G T C T G G C A C G A C G C T T G A A A A T T G T T C T C
RS-2       C C G C C C C G T C T G G C G C - A C G C T T G A A A A T T G T T C T C

RS-1(a)    G G G C T G C G A C G G A A C C A C G C G - T C C G A T G T C G C G G C
RS-1(b)    G G G C T G C G A C G G A A C C A G C C G G T C C G A T G T C G C G - -
RS-2       G G G C C G C G A C G G A A G C A - - C G A C T G C C A G T C G C G G C

RS-1(a)    A T C C C C T G G G T C G A T T C G A A C A C G A A A A G G A A G A A A
RS-1(b)    A T C A - C T C G G T C G G T T C G A - C A C G A A A A G G A A G C A A
RS-2       A T C A C C T C G G T C G G T T C G A A C A C G A A A A G G A A G C A A

                   Met   Ala   Ala   Leu   Arg   Gln   Ile   Ala   Phe   Tyr   Gly
RS-1(a)    T A     A T G G C T G C T C T G C G T C A G A T C G C G T T T T A C G G A
                         Leu
RS-1(b)    T A     A T G C T T G C T C T G C G T C A G A T C G C G T T T T A C G G A
                         Ala
RS-2       T A     A T G G C T G C T C T C C G T C A G A T C G C G T T T T A C G G A

            Lys   Gly   Gly   Ile   Gly   Lys   Ser   Thr   Thr   Ser   Gln   Asn
RS-1(a)    A A G G G A G G C A T T G G C A A A T C C A C T A C A T C C C A G A A T
RS-1(b)    A A G G G A G G C A T T G G C A A A T C C A C T A C A T C C C A A A A T
RS-2       A A G G G A G G C A T T G G C A A A T C C A C T A C A T C C C A A A A T

            Thr   Leu   Ala   Ala   Leu   Val   Glu   Leu   Gly   Gln   Lys   Ile
RS-1(a)    A C G C T C G C T G C C C T C G T C G A A C T T G G G C A G A A A A T C
RS-1(b)    A C G C T C G C T G C C C T T G T C G A A C T T G G G C A G A A A A T C
                  Pro
RS-2       A C G C C C G C C G C C C T T G T C G A A [.......not sequenced.....]

            Leu   Ile   Val   Gly   Cys   Asp   Pro   Lys
RS-1(a)    C T C A T C G T C G G C T G C G A C C C A A A A..................
RS-1(b)    C T C A T C G T C G G C T G C G A C C C A A A G [....not sequenced.....]
```

The experiments which provided the above data on the nucleotide sequences of RS-1(a), RS-1(b), RS-2 and RS-3 were repeated and extended. The results from these repeated experiments are given below as nucleotide sequences (Table 2) and as the predicted amino acid sequences from the open reading frames (Table 3). The codon (ATG) initiating the open reading fram (i.e., the first methionine residue; Table 3) is indicated in Table 2 as an enclosed box. The sequences shown in Table 2 are continuous from end to end. However, in order to render the homologies more readily discernible and to keep totally homologous regions in register, gaps have been left in some sequences. It will be understood that such gaps are not intended to reflect physical discontinuities.

Table 2

```
RS-2                   GATCTGCTTGCGCGCCTCTTCGGACTGCAGAAAGTTAACAAAACATGAGA
RS-3        CCGGTAAACCATAGCCATCCAGTCTCGCTAGAGAACAGCGCCGCTTTTCAAGGCCAGCAT

RS-1(a)                                ATTGCTGCTCGATTGG CGCTTCTACTCCGCG
RS-1(b)       ATCGATGCGCGGCTCATTCGACCGAGAGTTGTAGCGCATTGA AGCCGACCCACTGCT
RS-2        CTGAAGATGCTCAAGGGATAATCGCGCGCTTCGAAAATGTTTGCGACAGGCCCCTCTGCT
RS-3        TTGCATCAAGTGCGCTCGATCGACCGAGAGGTGCGTCATTGG GGCTCGCCCTCTGCGGC

RS-1(a)     GCCTCTTCAGGAGC     GACAGATGT  GACCAGTTGTCGTCACCTTTGTCGGCTTCGTG
RS-1(b)     TTCTCTTCAGGAGCAACATGACATGTGTCCGACA  TTGTCGTTTCCTTTGTCGACTTCGAG
RS-2        GTCTCTTCAGGATACTCACGACATGTGTGCGACA  TTGTCGTCAACTTTGTCGACTTCGTG
RS-3        T  TCTTCAGGGGCACCATGACATATGTGCGACA  TTGTCGT CGCTTTGTCGGCTTCGTG

RS-1(a)     ACACGCTTTAGGATTCTTCGGTCCGGTATTTTATCCCTCTAAGTGTCTGCGGCAGCACCAA
RS-1(b)     ACACGTCTTAGGATTCTTCGGTCCGATATTTTATCCCTCTAAGTGTTTGCGGCA CACCAA
RS-2        ACACGTCTTAGGATTCTTCGGTCCGGTATTTTATCCCTCTAAGTGTTTGCGGCAGCGCCAA
RS-3        ACACGGTTTAGGATTCTTCGGTCCAGTATTTTAAACCTCTAAGTGTCTGTGGCAGCACCAA

RS-1(a)     CTTCCGTTCTGCCCCTTCAATCAGCTCAAT TGGCACGACGCTTGAAAATTGTTCTCGGGC
RS-1(b)     ATTCCGTTCTGCCACATCAATCCGCCCAGTCTGGCACGACGCTTGAAAATTGTTCTCGGGC
RS-2        ATTCCGTTCTGCCACATCAATCCGCCCAGTCTGGCACGACGCTTGAAAATTGTTCTCGGGC
RS-3        CTTTCGTTCTGCCTA TCAATCAGCTCAAT TGGCACGACGCTTGAAAATTGTTCACGGGC

RS-1(a)     TGCGACGGAACCA  CGCGTCCGATGTCGCGGCATCCCCTCGGTCGATTCGAACACGAAAA
RS-1(b)     TGCGACGGAACCAGCCGCGTCCGATGTCGCGGCATCACCTCGGTCGGTTCGAACACGAAAA
RS-2        CGCGACGGAACCA  CGCGGCCGA GTCGCGGCATCACCTCGGTCGGTTCGAACACGAAAA
RS-3        TGCGACGGAGCCGGCTTGTATGAAACGGAGATCAGCATGTCCATCCGAAAAA    GAAAG
```

| | |
|---|---|
| RS-1(a) | GGAAGAAATAATGGCTGCTCTGCGTCAGATCGCGTTTTACGGAAAGGGAGGCATTGGCAAA |
| RS-1(b) | GGAAGCAATAATGGCTGCTCTGCGTCAGATCGCGTTTTACGGAAAGGGAGGCATTGGCAAA |
| RS-2 | GGAAGCAATAATGGCTGCTCTGCGTCAGATCGCGTTTTACGGAAAGGGAGGCATTGGCAAA |
| RS-3 | TTCTTCGTCAAGCGGGGACAAGGCATCGTGTTTGGCAGCTTCAACGGAACTAAAACTGCGGAGACAAA |

| | |
|---|---|
| RS-1(a) | TCCACTACATCCCAGAATACGCTCGCTGCCCTCGTC GAACTTGGGCAGAAAATCCTCATCG |
| RS-1(b) | TCCACTACATCCCAAAATACGCTCGCTGCCCTTGTC GAACTTGGGCAGAAAATCCTCATCG |
| RS-2 | TCCACTACATCCCAAAATACGCCCGCCGCCCTTGTC GAACTTGGGCAGAAAATCCTCATCG |
| RS-3 | CATGGAACCGGGTTTGAAAGCAGCGCT CCTTGATCAGATCT |

| | |
|---|---|
| RS-1(a) | TCGGCTGCGACCCAAAAGCTGA TTCGACGCGATTGATCCTGAACTCCAAAGCGCAGGGCAC |
| RS-1(b) | TCGGCTGCGACCC AAAGGCTGA TTCGACGCGATTGATTCTGAACTCCAAAGCGCAGGGCAC |
| RS-2 | CCGACTGCGACCC AAAGACCGAGTTCACGCAATTGATCCTGTTTGCGAAAAAGAAAAAAGC |
| RS-3 | |

| | |
|---|---|
| RS-1(a) | GGTTCTGGATCTAGCCGCAACGAAGGGTTCAGTTGAAGATCT GGAACTCGGCGATGTGCTC |
| RS-1(b) | TGTTCTGGATCTAGCCGCAACGAAGGGTTCAGTCGAAGATCT GGAACTCGGCGACGTGCTC |
| RS-2 | ATCCTTCGCAAAGCTC AAGGACCAAAACATTTGCATTTGGGAATTC |

| | |
|---|---|
| RS-1(a) | AAAACTGGCTACGGCGGCATCAAATGTGTGGAGTCGGGCGGCCCTGAACCCGGCGTCGGCT |
| RS-1(b) | AAAACTGGCTACGGCGGCATCAAATGTGTGGAGTCGGGCGGCCCTGAACCTGGCGTCGGCT |

| | |
|---|---|
| RS-1(a) | GCGCCGGACGGGGGGTCATAACGTCGATCAACTTCTTGGAAGAAAACGGCGCCTACGACGA |
| RS-1(b) | GCGCCGGACGGGGCGTCATCACATCGATCAACTTCCTGGAAGAAAACGGCGCCTATGACGA |

| | |
|---|---|
| RS-1(a) | TGTCGAC |
| RS-1(b) | CGTCGAC |

Table 3

| | |
|---|---|
| RS-1(a) | Met Ala Ala Leu Arg Gln Ile Ala Phe Tyr Gly Lys Gly Gly |
| RS-1(b) | Met Ala Ala Leu Arg Gln Ile Ala Phe Tyr Gly Lys Gly Gly |
| RS-2 | Met Ala Ala Leu Arg Gln Ile Ala Phe Tyr GLy Lys Gly Gly |
| RS-3 | Met Gly Pro GLy Leu Lys Ala Ala Leu Leu Asp Gln Ile |
| | |
| RS-1(a) | Ile Gly Lys Ser Thr Thr Ser Gln Asn Thr Leu Ala Ala Leu Val |
| RS-1(b) | Ile Gly Lys Ser Thr Thr Ser Gln Asn Thr Leu Ala Ala Leu Val |
| RS-2 | Ile Gly Lys Ser Thr Thr Ser Gln Asn Thr Pro Ala Ala Leu Val |
| | |
| RS-1(a) | Gly Leu Gly Gln Lys Ile Leu Ile Val Gly Cys Asp Pro Lys Ala Asp |
| RS-1(b) | Gly Leu Gly Gln Lys Ile Leu Ile Val Gly Cys Asp Pro Lys Ala Asp |
| RS-2 | Gly Leu Gly Gln Lys Ile Leu Ile Ala Asp Cys Asp Pro Lys Thr Glu |
| | |
| RS-1(a) | Ser Thr Arg Leu Ile Leu Asn Ser Lys Ala Gln Gly Thr Val Leu |
| RS-1(b) | Ser Thr Arg Leu Ile Leu Asn Ser Lys Ala Gln Gly Thr Val Leu |
| RS-2 | Phe Thr Gln Leu Ile Leu Phe Ala Lys Lys Lys Lys Ala Ser Phe |
| | |
| RS-1(a) | Asp Leu Ala Ala Thr Lys Gly Ser Val Glu Asp Leu Glu Leu Gly Asp |
| RS-1(b) | Asp Leu Ala Ala Thr Lys Gly Ser Val Glu Asp Leu Glu Leu Gly Asp |
| RS-2 | Ala Lys Leu Lys Asp Gln Asn Ile Cys Ile Trp Glu Phe |
| | |
| RS-1(a) | Val Leu Lys Thr Gly Tyr Gly Gly Ile Lys Cys Val Glu Ser Gly Gly |
| RS-1(b) | Val Leu Lys Thr Gly Tyr Gly Gly Ile Lys Cys Val Glu Ser Gly Gly |
| | |
| RS-1(a) | Pro Glu Pro Gly Val Gly Cys Ala Gly Arg Gly Val Ile Thr Ser Ile Asn |
| RS-1(b) | Pro Glu Pro Gly Val Gly Cys Ala Gly Arg Gly Val Ile Thr Ser Ile Asn |
| | |
| RS-1(a) | Phe Leu Glu Glu Asn Gly Ala Tyr Asp Asp Val Asp |
| RS-1(b) | Phe Leu Glu Glu Asn Gly Ala Thr Asp Asp Val Asp |

It can be seen (Tables 1 and 2) that the nucleotide sequences of RS-1(a), RS-1(b), RS-2 and RS-3 are strongly conserved, substantially homologous and that they could be used to identify the symbiotic (Sym) plasmids of specific isolates of Rhizobium.

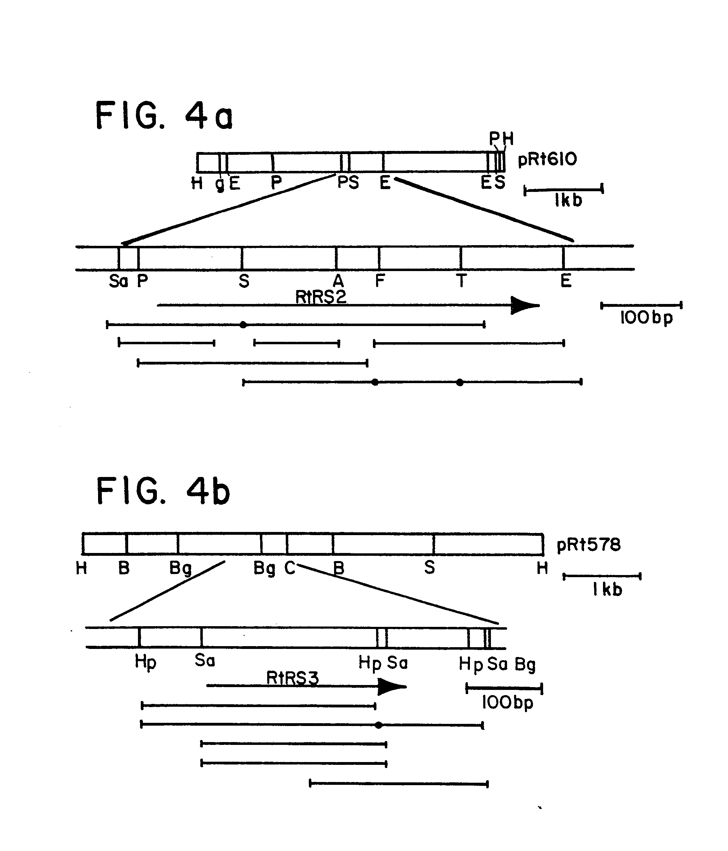

A number of other recombinant plasmids containing specific cloned fragments of nif DNA were constructed (Fig. 4). The nifH promoter was cloned in pRt642; the N-terminal end of the nifH coding region was cloned in pRt669 and a nifHD specific fragment was cloned in pRt680.

A Southern blot of total R. trifolii strain ANU843 DNA was hybridized sequentially with radioactively-labelled insert fragments from each of the above recombinant plasmids. When hybridized with the nifH promoter probe pRt642, five or six positively-hybridizing bands were observed indicating t at the repeated sequences is closely linked to, or is the nifH promoter. When the same blot was hybridized with the nifHD-specific probe pRt680, only a single positively-hybridizing fragment was observed, thus indicating that the DNA was completely digested and that only a single copy of the nifH and nifD genes was present in strain ANU843. Hybridization with the nifH-specific probe pRt669 showed that three copies of the N-terminal coding region of nifH were present.

The R. trifolii repeated sequences therefore fall into two classes. All copies are homologous with each other, but two of the sequences are linked to DNA homologous to the N-terminal end of the nifH structural gene. It is noteworthy that the repetitive sequences hybridize only within a Rhizobium species and do not hybridize between species.

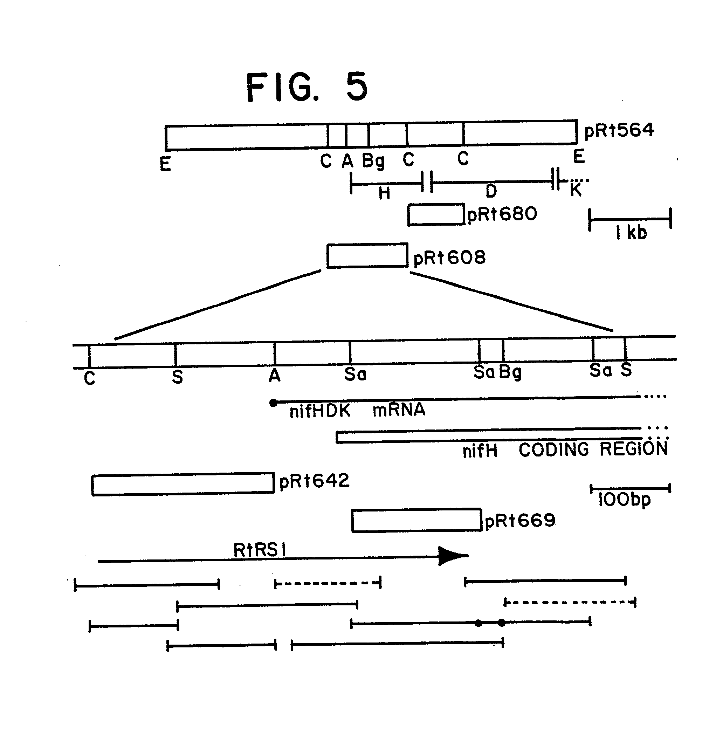

To locate other copies of the repeated sequence, the nifH promoter probe (pRt642) was used to screen a previously-constructed HindIII plasmid clone bank of R. trifolii ANU843 (Schofield, P. R. et al. (1983) Mol. Gen. Genet. 192:459-465). Two different recombinant plasmids were isolated from this bank. Restriction analysis showed that the plasmids contained inserts of 4.0 kb (pRt610) (Fig. 5a) and 6.0 kb in size and they were designated as containing the second (RS-2) and third (RS-3) copies of this repeated sequence. The recombinant plasmid containing RS-3 was shown by restriction analysis to be plasmid pRt578 (Fig. 5b), the insert of which is located approximately 30 kb to the right of the nifHDK genes on the ANU843 Sym plasmid (Schofield, P. R. et al. (1983) supra). The genes required for clover host-specific nodulation (Schofield, P. R. et al. (1984) Plant Mol. Biol. 3:3-11) are therefore flanked by two copies of the R. trifolii repeated sequence, namely RS-1(b) and RS-3.

The repeated sequence was further localized by Southern blot hybridization analysis of insert DNA of plasmids pRt610 and pRt578 and the RS-containing regions were sequenced. The restriction maps and DNA sequence analysis strategies used in determining the DNA sequence of RS-2 and RS-3 are shown (Fig. 5).

To determine the occurrence of the repeated sequence among R. trifolii isolates, a number of geographically distinct strains were examined. Total DNA was extracted from strains isolated in Australia, the Middle East, the United Kingdom and the United States.

The DNA was restricted with EcoRI, Southern blotted and hybridized with radioactively labelled RS-1(b), i.e., pRt642. Autoradiographs indicated that at least 3-6 copies of the repeated sequence were present in each of the R. trifolii isolates (Table 4). Each strain examined had a unique pattern of hybridizing fragments indicating the usefulness of the RDS probe, e.g., RS-1(b), in distinguishing between various R. trifolii strains. These results showed that the repeated sequence was highly conserved in all the R. trifolii isolates examined. Therefore, other fast-growing Rhizobium species were examined for the presence of this sequence. Total DNA was isolated from strains of R. trifolii, R. leguminosarum, R. meliloti, R. japonicum (a fast-growing species), Sesbania Rhizobium, and a fast-growing cowpea Rhizobium.

Southern blot hybridization analysis of DNA of these strains with radioactively labelled RS-1(b) (pRt642) resulted in hybridization only being detected with R. trifolii DNA and not with the DNA isolated from any other Rhizobium species. However, when the same Southern blot was rehybridized with the RS-1(b) and the nifH gene coding sequence (pRt608), positive hybridization to each of the Rhizobium DNA was observed. These hybridization data show that the R. trifolii repeated sequence, as exemplified by the R. trifolii RS-1(b) sequence, is unique to this species.

Conservation of the repeated sequence amongst geographically distinct R. trifolii isolates suggests that such sequences may play an important role in the ecology of this species. Indeed, the finding that the repeated sequences are located exclusively on the Sym plasmid, in the seven R. trifolii strains examined, further supports this hypothesis. Analogous repeated sequences in R. meliloti are plasmid located (Better, M. et al. (1983) Cell 35:479-485). However, some copies are located on a second plasmid and not on the Sym plasmid.

Hybridization experiments have demonstrated that RS-1(b) is specific for R. trifolii Ruvkun and Ausubel (Ruvkun, G. B. and F. M. Ausubel (1980) Proc. Nat. Acad. Sci. USA 77:191-195) have shown by hybridization analysis that the nitrogenase structural genes (nifDK) if diverse nitrogen-fixing microorganisms are highly conserved. However, the repeated sequences of the fast-growing Rhizobium species do not show a high degree of interspecific homology. Thus, while the structural genes which constitute the nifDK operon appear to hav been conserved in evolution, the repetitive sequences are evolutionarily divergent (at least among fast-growing, temperate legume-infecting species of Rhizobium). Considering the narrow plant host range shown by such fast-growing species, these observations suggest that species-specific repeated DNA sequences may be involved in host-specific expression of certain symbiotic genes.

In view of the species specificity of repetitive sequences, we suggest, but do not intend to be bound by, the following model for the activation of expression of nitrogen fixation genes in the fast-growing, temperate legume-infecting Rhizobium species. According to the model, the appropriate species-specific repeated sequence is required for expression of the *nif*DK genes (and other species-specific, repeated sequence activated genes) by the appropriate Rhizobium species which "normally" nodulates the given host plant. Thus, *R. trifolii* would only fix nitrogen in symbiosis with clovers if the *nif*DK and other repeated sequence-activated symbiotic genes carry the *R. trifolii* repeated sequences.

Under the conditions used in the present invention, the *R. trifolii* repeated sequences (RS) can be used as a repetitive diagnostic sequence (RDS) for both species and strain identification. A molecular approach to Rhizobium taxonomy obviates the possible deleterious effects induced by using antibiotic markers for strain identification (Schwinghamer, E. A. (1964) Can. J. Microbiol. 10:221-233; (1967) Antonie van Leeuwenhock 33:121-136). The presence of analogous, but species-specific, repeated DNA sequences in other fast-growing Rhizobium species will enable the rapid identificaiton of other Rhizobium species and unknown isolates.

Example 1: Isolation of a DNA fragment containing one or more nodulation genes.

Total R. trifolii DNA was prepared by harvesting 10ml of cells (O.D.$_{650}$ = 0.5) grown at 30°C with aeration in TY broth (Beringer, J. E. (1974) J. Gen. Microbiol. 84:188-198). The cell pellet was washed twice in 3ml of TES (10mM Tris, pH 8.0; 1mM EDTA; 100mM NaCl) and resuspended in 1ml of TE (10mM Tris, pH 8.0; 1mM EDTA). 0.2ml of fresh lysozyme (5mg/ml in TE), 0.4ml of 0.25 M EDTA were added and incubated at 37°C for 30 minutes. 0.5ml of pronase (1mg/ml in TE) and 0.1ml of 20% SDS were added and the digest was incubated at 37°C for 60 minutes. The digest was extracted four times with 4ml of phenol:chloroform (1:1). The two phases were separated by centrifugation (20,000 rpm, 20 minutes, SS-34 rotor). The DNA was precipitated with 2.5 vol. of -20°C ethanol and 0.1 vol. of 3M sodium acetate, pH6.0 at -70°C for 30 minutes. After centrifugation the DNA precipitate was redissolved in 1ml of TE and dialysed against three changes of TE buffer.

Following restriction with the appropriate endonuclease in TA buffer (O'Farrell, P. H., Kutter, E. and M. Nakanishi (1980) Molec. Gen. Genet. 179:421-435), DNA was electrophoresed in 1% horizontal TAE (40mM Tris; 20mM sodium acetate; 2mM EDTA, pH 7.8) agarose gels (4 x 140 x 190 mm) at 40 volts for 16-18 hours. After transfer to nitrocellulose (Southern, E. M. (1975) J. Molec. Biol. 98:503-517), the DNA was hybridized to $10^7$ cpm of an [$\alpha^{32}$P]dCTP-labelled probe prepared by the method of random priming (Whitfeld, P. L., Seeburg, P. H. and J. Shine (1982) DNA 1:133-143) at 65°C for 16 hours.

The probe used was pRt851 (a Rhizobium DNA fragment containing a Tn5 insertion from ANU851 cloned in the vector plasmid pBR322). Tn5 mutagenesis of R. trifolii wild type strain ANU843 has previously been used to isolate mutants defective in many of the steps leading to symbiotic nitrogen fixation (Scott, K. F., Hughes, J. E., Gresshoff, P. M., Beringer, J. E., Rolfe, B. G. and J. Shine (1982) J. Mol. Appl. Genet. 1:315-326). The majority of thes mutants can still initiate nodule development but are defective in one of he later steps of the symbiosis, such as release of bacteria from the infect n thread, bacteroid development or nitrogen fixation. The Tn5-induced mutar of R. trifolii ANU851 used as a probe in this example is a stable nodulation deficient (Nod$^-$) phenotype and is unable to induce clover root hair curli (Hac-), an early stage in nodule development (Rolfe, B. G., Djordjevic, M. A.,

Scott, K. F., Hughes, J. E., Badenoch-Jones, J., Gresshoff, P. M., Cen, Y., Dudman, W. F., Zurkowzki, W. and J. Shine (1981) in "Current Perspectives in Nitrogen Fixation", Gibson, A. H. and W. E. Newton, eds. pp. 142-145 Australian Academy of Science, Canberra). Southern blot analysis (Southern, E. M. (1975) J. Molec. Biol. 98:503-517) of DNA from the Hac⁻ mutant ANU851, showed the presence of a unique band of approximately 13kb (5.7kb of Tn5 inserted into a 7.2kb EcoRI restriction fragment) that hybridized to Tn5 sequences. This confirmed that a single Tn5 insertion was responsible for the Hac⁻ phenotype of ANU851. The 13kb DNA fragment containing Tn5 and flanking *R. trifolii* sequences was isolated from ANU851 by restriction of total DNA with EcoRI and molecular cloning into the *Escherichia coli* plasmid pBR322 to give the recombinant plasmid pRt851.

Example 2: Construction of clone banks of Rhizobium DNA.

Clone banks of *R. trifolii* strain ANU843 were constructed in the plasmid cloning vector pBR328 (Soberon, X., *et al*., (1980) Gene 9:287). Vector DNA was digested to completion with the restriction endonuclease EcoRI, HindIII or BamHI in TA buffer (O'Farrell, *et al*., (1980) Molec. Gen. Genet. 179:421-435). After inactivating the restriction enzymes by phenol:chloroform (1:1) extraction, the DNA was ethanol precipitated, pelleted by centrifugation and dried *in vacuo*. The restricted vector DNA was reconstituted in 0.1M Tris base, pH 10-11, containing 0.2% sodium dodecyl sulfate and 0.1-0.2 units of calf intestinal alkaline phosphatase per μg of DNA. The reaction mixture was incubated at 37°C for one hour, extracted three times with an equal volume of phenol: chloroform (1:1), ethanol precipitated, dried *in vacuo* and reconstituted at a concentration of 500 μg/ml in TE buffer.

Total DNA (5-10 μg), derived from *R. trifolii*, was digested to completion with EcoRI, HindIII or BamHI in TA buffer. Reactions were terminated by heating the digests at 65°C for 15 minutes. The DNA was then ethanol precipitated, dried and reconstituted in a final volume of 20-50 μl containing 10mM Tris, pH 7.4, 8mM $MgCl_2$, 10mM β-mercaptoethanol, 1mM ATP and 0.5 μg of appropriately-digested, alkaline phosphatase-treated pBR328 DNA. After the addition of 5 units of T4 DNA ligase, the mixture was incubated at 20-25°C for 3-4 hours.

Ligated DNA (equivalent to 100-200ng of pBR328) was diluted to 100 µl with TE buffer and transformed into 200 µl of preserved competent cells of Escherichia coli strain RR1 (Morrison (1977) J. Bacteriol. 132:349).

Transformants were selected on L agar (Miller, (1972) Experiments in Molec. Biol., Cold Spring Harbor Lab, New York) containing 50µg of ampicillin per ml. The frequency of recombinant plasmids was assessed by spotting 100-200 transformant clones to L agar containing 50µg of chloramphenicol per ml (for EcoRI clones) or 25µg of tetracycline per ml (for HindIII or BamHI clones).

Generally, 90-95% of the transformants contained recombinant plasmids as indicated by insertional inactivation (Timmis, et al., (1974) Proc. Nat. Acad. Sci. U.S.A. 71:4556).

Example 3: Isolation of wild type nod genes

Wild-type ANU843 DNA was cleaved with various restriction endonucleases and cloned as described in Example 2, and the hybridization probe described in Example 1 were used to detect wild-type nod genes. When pRt851 was used as the hybridization probe and EcoRI as the restriction endonuclease, a 7.2kb wild-type EcoRI restriction fragment was recovered. This was cloned into pBR328 to yield recombinant plasmid pRt572. If HindIII was used as the restriction enzyme, a 14kb wild type HindIII restriction fragment was recovered. This was also cloned into pBR328 to yield recombinant plasmid pRt587. These two recombinant plasmids (pRt572 and pRt587) were then used as probes in a "walk" to detect overlapping recombinant clones derived by cleavage with a different restriction endonuclease. In this manner, various fragments covering 27kb DNA spanning all the nif and nod genes and the adjacent flanking sequences were recovered and mapped as shown in Fig. 1. The various fragments were isolated by cloning into the vector plasmid pBR328 (Soberon, X., Covarrubias, L., and F. Bolivar (1980) Gene 9:287-305) and transformed into Escherichia coli RRI.

In Fig. 1, H, B and E represent cleavage sites for HindIII, BamHI and EcoRI, respectively. The nif H and D genes of ANU843 are shown, together with arrows indicating the direction of transcription of these genes. Horizontal bars indicate the regions carried by the respective recombinant plasmids. Two RDS's are shown as circles on the map, designated RS1 and RS3. These are seen to be included within the fragments pRt585 and pRt578, respectively.

Example 4: Preparation of hybridization probes

Radioactive probes were prepared by primed synthesis using random 8-12 nucleotide long oligonucleotide primers made from calf thymus DNA. Template DNA (100-200 ng purified restriction fragment, or 1-2μg linear plasmid DNA) and 100μg primer were mixed in 20μl $H_2O$, denatured by boiling for 2 minutes and quick cooled on ice. Synthesis was initiated by the addition of 50mM Tris-HCl pH 8.0, 20mM KCl, 7mM $MgCl_2$, 10mM β-mercaptoethanol, 600μM dGTP, 600μM dTTP, 600μM dATP, 0.3μM $\alpha^{32}$P-dCTP (3000 Ci/mmole, Amersham) and 5 units E. coli DNA polymerase I (Klenow fragment). This mix was incubated at 37°C for 30 minutes. For probes with higher specific activities the 600μM dATP was replaced with 0.3μM $\alpha^{32}$P-dATP (3000 Ci/mmole). The reaction was stopped with 25mM EDTA and extracted with phenol and chloroform.

To separate the unincorporated nucleotides from $^{32}$P-labelled DNA, the aqueous phase was loaded onto a Sephadex G-50 column (5cm x 9mm) and washed through with TEN buffer (0.1M NaCl, 10mM Tris-HCl pH 8.0 and 1mM EDTA). Fractions (3-4 drops, 200-500μl) were collected and the void fractions containing radioactively labelled DNA (as monitored by a Geiger counter-minimeter) were pooled. E. coli tRNA (30μg) was added as a carrier and the DNA was recovered by ethanol precipitation. Probes were denatured by boiling for 2 minutes before use. Specific activities obtained generally ranged from $1 \times 10^7$ to $9 \times 10^7$ cpm/μg template DNA.

Example 5: Detection of repetitive sequences in the Sym plasmid.

When certain of the fragments cloned into the vector plasmid pBR328, e.g., recombinant plasmids pRt585 and pRt578 (Fig. 1; also see Example 2), were radioactively labelled and used as hybridization probes against restricted wild-type ANU843 DNA, it was found that they hybridized to several different bands, thus demonstrating repetitive sequences. These complementary fragments were not located on the bacterial chromosome because no complementation occurred when the hybridization test was done with similarly-restricted DNA from ANU845. ANU845 is a derivative of ANU843 and differs from that strain by the loss of the Sym plasmid, i.e., it has been "cured" of the plasmid. These recombinant plasmids (pRt585 and pRt578) were amplified and purified. They were then radioactively labelled and used as probes to test for the presence of the symbiotic (Sym) plasmid in other isolates of R. trifolii and other species such as R. meliloti and R. leguminosarum.

Example 6: Detection of the Sym plasmid and of RDS in isolates of Rhizobia.

The strain to be tested was grown in culture and the DNA was isolated. Alternatively the DNA from the various plasmids was isolated and the Sym plasmid was purified (Hooykaas, P. J. J., van Brussell, A. A. N., den Dulk-Ras, H., van Slogteren, G. M. S. and R. A. Schilperoort (1981) Nature 291:351). The DNA to be tested was then suitably restricted and the fragments separated by agarose gel electrophoresis (Aaij, C. and P. Borst (1972) Biochem. Biophys. Acta. 269:503-517). The electrophoresed DNA fragments were then transferred to nitrocellulose filters (Schleicher and Schull, BA85) (Lawn, R. M., Fritsch, E. F., Parker, R. C., Blake, G. and T. Maniatis (1978) Cell 15:1157-1174) and the double stranded DNA probes (e.g., pRt585 or pRt578) were radioactively labelled by nick translation (Rigby, P. W. J., Dieckmann, M., Rhodes, C. and P. Berg (1977) J. Mol. Biol. 113:237-251. It will be readily apparent to those skilled in the art that if the DNA to be tested and the probe were obtained by use of the same restriction enzyme, then a minimum value for the number of RDS in a symbiotic (Sym) plasmid can be determined.

The nitrocellulose filters containing immobilized Sym plasmid DNA restriction fragments were prehybridized for two hours at 65°C in 3 x SSC (0.45 M NaCl, 0.045 M Na-citrate), 50mM HEPES pH 7.0 (N-2-hydroxyethylpiperazine-N'-2-ethanesulfonic acid), 0.1% (w/v) $NaDodSO_4$ (sodium dodecyl sulfate), 0.2% (w/v) each polyvinylpyrrolidone (PVP), Ficoll, bovine serum albumin (BSA), 20 μg/ml denatured and sheared herring sperm DNA, and 20 μg/ml E. coli tRNA before addition of approximately $1 \times 10^6$ cpm probe DNA per filter. Hybridization was carried out for 18-24 hours at 65°C. Filters were washed in 2 x SSC and 0.1% $NaDodSO_4$ at room temperature for two hours before exposing to X-ray film (Kodak XS-5) in the presence of an intensifying screen (Dupont Cronex lightning plus) at -70°C for 1-3 days. If RDS are present on the Sym plasmid or the restricted DNA, then more than one band will "light up" on the autoradiograph.

When labeled pRt585 or pRt578 were used as probes, multiple bands of Sym plasmid DNA were labeled, indicating that these probes included a common sequence (RDS). The multiple bands were more readily observable at longer exposure times. Multiple bands were not observed when other probes (e.g., pRt572) were used, nor were multiple bands observed when the RDS-containing probes were hybridized to Sym plasmid DNA from other Rhizobial species, e.g. R. meliloti.

Example 7: Construction of a probe containing RS-1(b)

Digestion of the Sym plasmid of Rhizobium trifolii with the restriction enzyme ClaI yielded a DNA fragment which comprises the RS-1(b) RDS and the nifH gene. The ends of this fragment occurred 50-60 base pairs to the left c RS-1(b) and at an undefined distance to the right of the nifH gene (Fig. 3). This Sym plasmid ClaI fragment was inserted into the ClaI site of pBR 328. Insertion may occur in either orientation, and insertions in both orientatior were recovered.

The Sym plasmid ClaI fragment contained an AvaI site immediately to the left of the nifH gene (Fig. 3) and pBR328 contains a single AvaI site. The recombined pBR328 plasmid (i.e., pBR328 + the Sym plasmid ClaI fragment) was cleaved with AvaI, a different DNA fragment being deleted depending on the insertion orientation. In one orientation the RS-1(b) sequence was deleted (pRt608 - Fig. 3) while in the other orientation the nifH gene was deleted (pRt642 - Fig. 3). Following deletion of the nifH gene, the recombinant plasmid pRt642 contained RS-1(b), about 50-60 bp to the left of RS-1(b) and about 200 bp to the right of RS-1(b) (Fig. 3). Recombinant plasmid pRt642 would then be used as a probe to more precisely locate the positions of the RDS segments on any symbiotic plasmid of any Rhizobium isolate.

Example 8: A general method for the isolation of a Rhizobium diagnostic segment (RDS)

An RDS is defined as a member of a family of substantially homologous DN/ sequences specific to a given Rhizobium species. In turn, a substantially homologous DNA sequence is defined in terms of the conditions which permit hybridization of denatured DNA fragments as described in this example (see below).

The presence of an RDS is detected when a cloned DNA fragment (see Example 2), of given size, is radioactively labelled and hybridized to restriction fragments of total DNA (cut with the same enzyme as was used for cloning). Normally only one positively-hybridizing fragment, namely that corresponding to the cloned probe fragment will be seen. However, if the probe includes an RDS, then additional positively-hybridizing bands will be seen.

Further proof for the presence of an RDS is obtained by subdividing the original fragment into smaller subfragments with a second restriction enzyme. When each of the subfragments is radioactively labelled and separately probed onto restriction fragments of total DNA (generated by the original cloning enzyme), then each will hybridize to the original cloned fragment. However, one or more of the subfragments will also hybridize to the multiplicity of fragments observed when the entire fragment is used as a probe. In this way the RDS can be mapped and probes prepared which are mostly RDS with a minimum of flanking DNA.

It will be noted from this description that repeated sequences are not cloned directly but are found on fragments which have already been cloned. In this case, the cloned fragments were obtained from a plasmid clone bank of Rhizobium trifolii strain ANU843 DNA fragments (see Example 2).

More specifically, (see Fig. 2) total DNA was isolated from eight independent isolates of Rhizobium trifolii (tracks 2-9) and one strain of R. meliloti (track 10) by the method of Schofield et al. (1983, Mol. Gen. Genet. In press). The resultant total DNA's were restricted completely with the restriction endonuclease EcoRI in TA buffer (O'Farrell, et al. (1980) Molec. Gen. Genet. 179:421-435) and electrophoresed in 1% horizontal TAE (40mM Tris:20mM sodium acetate:2mM EDTA, pH 7.8) agarose gels (4 x 140 x 190mm) at 40 volts for 16-18 hours. DNA fragments were transferred to nitrocellulose (Southern, E. M. (1975) J. Molec. Biol. 98:503-517) and hybridized to $10^7$ cpm of an $\alpha$-$^{32}$P-dCTP labelled probe (pRt607) prepared by the method of random priming (Whitfeld, P. L., et al. (1982) DNA 1:133-143) at 65°C for 16 hours. It will be understood by those skilled in the art that temperatures lower than 65° and/or times longer than 16 hours will decrease the stringency of the hybridization conditions and that the length and degree of complementarity of the DNA under consideration will also affect the extent of hybridization. RDS from different locations on the same Sym plasmid are sufficiently self-complementary to hybridize under the described conditions and under less stringent conditions. However, under less stringent conditions, the likelihood of false positive results increases. The recombinant plasmid, pRt607 (Fig. 3 and Example 7), is a derivative of pBR328 (Soberon, X., et al., supra) which contains a 1kb ClaI fragment, derived from the Sym plasmid of R. trifolii strain ANU843, on which the repeated sequence RS-1(b) and part of the nifH structural gene are located. The nitrocellulose was washed three times for 15 minutes in 2 x SSC (standard saline citrate) at 65°C and exposed to X-ray film (Kodak XAR-5) in the presence of an intensifying screen (Dupont Cronex Lightning Plus) at -70°C for 2 days. The results are shown in Fig. 2. Tracks 1 and 11 show HindIII fragments of bacteriophage lambda with sizes expressed in kilobases.

Alternatively, the probe pRt607 can be made smaller and more specific by cleaving with the restriction enzyme AvaI, which will remove the nifH portion of the probe and a non-essential DNA sequence of pBR328. Following religation, a plasmid designated pRt642 comprising the repeated sequence RS-1(b) is recovered. This more specific probe pRt642 can be used as described above to detect substantially homologous RDS.

Example 9: Isolation of RDS of the symbiotic (Sym) plasmid of Rhizobium meliloti.

Cells of R. trifolii are grown and harvested as described in Example 1. Then the DNA from the various plasmids is isolated and the Sym plasmid is purified (Hooykaas, P. J. J., van Brussell, A. A. N., den Dulk-Ras, H., van Slogteren, G. M. S. and R. A. Schilperoort (1981) Nature, London 291:351). The DNA to be tested is then suitably restricted, e.g., by EcoRI, and the fragments are separated by agarose gel electrophoresis (Aaij, C. and P. Borst (1972) Biochim. Biophys. Acta. 269:503-517). The electrophoresed DNA fragments are transferred to nitrocellulose filters (Schleicher and Schull, BA85) (Lawn, R. M., Fritsch, E. F., Parker, R. C., Blake, G. and T. Maniatis (1978) Cell 15:1157-1174).

The identical DNA which is to be tested (see previous paragraph) is then again restricted with the same enzyme, i.e., EcoRI, and a number of the fragments are cloned into a suitable vector, e.g., pBR322, transformed into a suitable host, e.g., E. coli HB101, and amplified. Following amplification, the plasmid is purified and the inserted fragment excised with EcoRI. These double stranded DNA fragments are then radioactively labelled by nick translation (Rigby, P. W. J., Dieckmann, M., Rhodes, C. and P. Berg (1977) J. Mol. Biol. 113:237-251) and used as probes to detect substantially homologous RDS separated by electrophoresis and transferred to nitrocellulose filters as described in the previous paragraph. Hybridization conditions are the same as described in Example 8. It will be obvious that the probe is homologous to at least the one corresponding fragment of the DNA to be tested since both are digested by the same restriction enzyme. If the DNA in the probe represents a unique sequence, then only one band will "light up" following hybridization and autoradiography; whereas if the probe comprises an RDS, then a number of bands corresponding to the various RDS of that symbiotic (Sym) plasmid will light up. In the latter case, the cloned probe fragment is maintained and can be used to detect substantially homologous RDS from Rhizobium isolates obtained from soil samples or from root nodules. The procedure is repeated with other restriction enzymes, giving similar multiple bands in response to a probe comprising RDS. Control hybridizations using an RDS probe of another Rhizobium species (e.g., pRt642) demonstrates lack of substantial homology between RDS of the two species. It will be understood that a control probe from another species may hybridize with one of the DNA fragments if the probe is large enough to include sequences flanking the RDS that are conserved between the species. Such a result was observed when pRt585 was used to probe R. meliloti DNA, as shown in Fig. 2, lane 10. In that instance, the presence of part of nifH flanking RS-1(b) resulted in hybridization, since the nifH gene is known to be highly conserved.

The foregoing method is generally applicable to the isolation of RDS from any Rhizobium species.

Example 10: Rhizobium species determination using RDS probes.

Applying the techniques of the foregoing Example 9 to the cloning of RDS probes specific for each Rhizobium species enables the preparation of a battery of species-specific RDS probes. Soil isolates of Rhizobia are grown under laboratory culture conditions from single-colony isolates, using techniques known to those of ordinary skill in the art. The DNA of each strain is isolated and fragments thereof are obtained with the use of a restriction endonuclease, essentially as described in Example 8. The DNA fragments are fractionated by agarose gel electrophoresis as described in Example 8 and probed by hybridization with a species-specific RDS probe, labelled as described in Example 4.

A positive identification of the unknown strain as belonging to a given Rhizobium species will be made in the instance where an RDS probe from that species labels ("lights up") more than one band of fractionated DNA. When DNA of the unknown strain is probed with an RDS probe of a different species, there may be no hybridization of the probe to any DNA fragment or, there may be hybridization to one band if the RDS probe happens to include a region of homology with the unknown DNA flanking the RDS. It will be understood that more definitive test results will be obtained where the RDS probe includes a minimum of such flanking DNA.

The following strains and plasmids were deposited at the American Type Culture Collection, 12301 Parklawn Drive, Rockville, Maryland 20852.

| | Plasmid or Strain | Deposit Date | ATCC No. |
|---|---|---|---|
| 1. | Escherichia coli RR1/pRt578 | 12/21/83 | 39545 |
| 2. | Escherichia coli RR1/pRt607 | 12/21/83 | 39546 |
| 3. | Escherichia coli RR1/pRt608 | 12/21/83 | 39547 |
| 4. | Escherichia coli RR1/pRt610 | 12/21/83 | 39548 |
| 5. | Escherichia coli RR1/pRt642 | 12/21/83 | 39549 |
| 6. | pRt578 (RS-3) | 12/21/83 | 40089 |
| 7. | pRt607 (RS-1(b)) | 12/21/83 | 40090 |
| 8. | pRt608 (RS-1(b)) | 12/21/83 | 40091 |
| 9. | pRt610 (RS-2) | 12/21/83 | 40092 |
| 10. | pRt642 | 12/21/83 | 40093 |

What is claimed is:

1. A bacterial strain containing and replicating therein a recombinant DNA plasmid comprising;

   (a) a vector and

   (b) a RDS derived from a symbiotic *Sym* plasmid recovered from a nitrogen fixing bacterial species.

2. A bacterial strain as recited in Claim 1 wherein said strain is *Escherichia coli* RRI.

3. A bacterial strain as recited in Claim 1 wherein said vector is pBR328.

4. A bacterial strain as recited in Claim 1 wherein said RDS is derived from a symbiotic *Sym* plasmid of a *Rhizobium* isolate.

5. A bacterial strain as recited in Claim 1 wherein said RDS is derived from a symbiotic *Sym* plasmid of *Rhizobium trifolii*.

6. A recombinant DNA plasmid comprising a RDS derived from a symbiotic *Sym* plasmid and a vector.

7. A recombinant DNA plasmid as recited in Claim 6 wherein said vector is pBR328.

8. A recombinant DNA plasmid as recited in Claim 6 wherein said RDS is derived from a symbiotic *Sym* plasmid of *Rhizobium trifolii*.

9. A recombinant DNA plasmid as recited in Claim 6 wherein said RDS is derived from a symbiotic *Sym* plasmid of a *Rhizobium* isolate.

10. A RDS derived from a symbiotic *Sym* plasmid derived from a bacterial species.

11. A RDS as recited in Claim 10 wherein said RDS is derived from a symbiotic <u>Sym</u> plasmid of a <u>Rhizobium</u> isolate.

12. A RDS as recited in Claim 10 wherein said RDS is derived from a symbiotic <u>Sym</u> plasmid of <u>Rhizobium trifolii</u>.

13. A recombinant plasmid comprising an RDS, said RDS being obtained from a symbiotic <u>Sym</u> plasmid of a <u>Rhizobium</u> species and located at more than one location thereon, said RDS comprising a sequence of at least 100 nucleotides, said RDS being substantially homologous with a first RDS obtained from any <u>Rhizobium</u> strain of the same species but being non-homologous with a second RDS obtained from a <u>Rhizobium</u> strain of a different species.

14. A recombinant plasmid according to Claim 13 wherein said RDS is obtained from <u>Rhizobium trifolii</u>, the RDS thereof comprising any of the sequences RS-1(a), RS-1(b), RS-2 or RS-3 or being substantially homologous therewith.

15. A recombinant plasmid according to Claim 13 wherein said RDS is obtained from <u>Rhizobium trifolii</u>, the RDS thereof comprising the sequence 5'-A-T-T-G-C-T-G-C-T-C-G-A-T-T-G-G-C-G-C-T-T-C-T-A-C-T-C-C-G-C-G-G-C-C-T-C-T-T-C-A-G-G-A-G-C-G-A-C-A-G-A-T-G-T-G-A-C-C-A-G-T-T-G-T-C-G-T-C-A-C-C-T-T-T-G-T-C-G-G-C-T-T-C-G-T-G-A-C-A-C-G-C-T-T-T-A-G-G-A-T-T-C-T-T-C-G-G-T-C-C-G-G-T-A-T-T-T-T-A-T-C-C-C-T-C-T-A-A-G-T-G-T-C-T-G-C-G-G-C-A-G-C-A-C-C-A-A-C-T-T-C-C-G-T-T-C-T-G-C-C-C-C-T-T-C-A-A-T-C-A-G-C-T-C-A-A-T-T-G-G-C-A-C-C-A-C-G-C-T-T-G-A-A-A-A-T-T-G-T-T-C-T-C-G-G-G-C-T-G-C-G-A-C-G-G-A-A-C-C-A-C-G-C-G-T-C-C-G-A-T-G-T-C-G-C-G-G-C-A-T-C-C-C-C-T-G-G-G-T-C-G-A-T-T-C-G-A-A-C-A-C-G-A-A-A-A-G-G-A-A-G-A-A-A-T-A-A-T-G-G-C-T-G-C-T-C-T-G-C-G-T-C-A-G-A-T-C-G-C-G-T-T-T-T-A-C-G-G-A-A-A-G-G-G-A-G-G-C-A-T-T-G-G-C-A-A-A-T-C-C-A-C-T-A-C-A-T-C-C-C-A-G-A-A-T-A-C-G-C-T-C-G-C-T-G-C-C-C-T-C-G-T-C-G-A-A-C-T-T-G-G-G-C-A-G-A-A-A-A-T-C-C-T-C-A-T-C-G-T-C-G-G-C-T-G-C-G-A-C-C-C-A-A-A-A-G-C-T-G-A-T-T-C-G-A-C-G-C-G-A-T-T-G-A-T-C-C-T-G-A-A-C-T-C-C-A-A-A-G-C-G-C-A-G-G-G-C-A-C-G-G-T-T-C-T-G-G-A-T-C-T-A-G-C-C-G-C-A-A-C-G-A-A-G-G-G-T-T-C-A-G-T-T-G-A-A-G-A-T-C-T-G-G-A-A-C-T-C-3', or being substantially homologous therewith.

16. A recombinant plasmid according to Claim 13 wherein said RDS is obtained from <u>Rhizobium</u> <u>trifolii</u>, the RDS thereof comprising the sequence 5'-A-T-C-G-A-T-G-C-G-C-G-G-C-T-C-A-T-T-C-G-A-C-C-G-A-G-A-G-T-T-G-T-A-G-C-G-C-A-T-T-G-A-A-G-C-C-G-A-C-C-C-A-C-T-G-C-T-T-T-C-T-C-T-T-C-A-G-G-A-G-C-A-A-C-A-T-G-A-C-A-T-G-T-G-T-C-C-G-A-C-A-T-T-G-T-C-G-T-T-T-C-C-T-T-T-G-T-C-G-A-C-T-T-C-G-A-G-A-C-A-C-G-T-C-T-T-A-G-G-A-T-T-C-T-T-C-G-G-T-C-C-G-A-T-A-T-T-T-T-A-T-C-C-C-T-C-T-A-A-G-T-G-T-T-T-G-C-G-G-C-A-C-A-C-C-A-A-A-T-T-C-C-G-T-T-C-T-G-C-C-A-C-A-T-C-A-A-T-C-C-G-C-C-C-A-G-T-C-T-G-G-C-A-C-G-A-C-G-C-T-T-G-A-A-A-A-T-T-G-T-T-C-T-C-G-G-G-C-T-G-C-G-A-C-G-G-A-A-C-C-A-G-C-C-G-C-G-T-C-C-G-A-T-G-T-C-G-C-G-G-C-A-T-C-A-C-C-T-C-G-G-T-C-G-G-T-T-C-G-A-A-C-A-C-G-A-A-A-A-G-G-A-A-G-C-A-A-T-A-A-T-G-G-C-T-G-C-T-C-T-G-C-G-T-C-A-G-A-T-C-G-C-G-T-T-T-T-A-C-G-G-A-A-A-G-G-G-A-G-G-C-A-T-T-G-G-C-A-A-A-T-C-C-A-C-T-A-C-A-T-C-C-C-A-A-A-A-T-A-C-G-C-T-C-G-C-T-G-C-C-C-T-T-G-T-C-G-A-A-C-T-T-G-G-G-C-A-G-A-A-A-A-T-C-C-T-C-A-T-C-G-T-C-G-G-C-T-G-C-G-A-C-C-C-A-A-A-G-G-C-T-G-A-T-T-C-G-A-C-G-C-G-A-T-T-G-A-T-T-C-T-G-A-A-C-T-C-A-A-A-G-C-G-C-A-G-G-G-C-A-C-T-G-T-T-C-T-G-G-A-T-C-T-A-G-C-C-G-C-A-A-C-G-A-A-G-G-G-T-T-C-A-G-T-C-G-A-A-G-A-T-C-T-G-G-A-A-C-T-C-3', or being substantially homologous therewith.

17. A recombinant plasmid according to Claim 13 wherein said RDS is obtained from <u>Rhizobium</u> <u>trifolii</u>, the RDS thereof comprising the sequence 5'-C-T-G-C-A-G-A-A-A-C-T-G-A-A-G-A-T-G-C-T-C-A-A-G-G-G-A-T-A-A-T-C-G-C-G-C-G-C-T-T-C-G-A-A-A-A-T-G-T-T-T-G-C-G-A-C-A-G-G-C-C-C-C-T-C-T-G-C-T-G-T-C-T-C-T-T-C-A-G-G-A-T-A-C-T-C-A-C-G-A-C-A-T-G-T-G-T-G-C-G-A-C-A-T-T-G-T-C-G-T-C-A-A-C-T-T-T-G-T-C-G-A-C-T-C-G-T-G-A-C-A-C-G-T-C-T-T-A-G-G-A-T-T-C-T-T-C-G-G-T-C-C-G-G-T-C-C-G-G-T-A-T-T-T-T-A-T-C-C-C-T-C-T-A-A-G-A-G-T-T-T-G-C-G-G-C-A-G-G-C-C-A-A-A-T-T-A-C-G-T-T-C-T-G-C-C-C-A-C-A-A-T-C-A-A-T-T-G-C-C-C-A-G-T-C-T-G-G-C-A-C-G-A-C-G-C-T-T-G-A-A-A-A-T-T-G-T-T-C-T-C-G-G-G-C-C-G-C-G-A-C-G-G-A-A-A-C-A-A-A-C-A-C-G-T-T-C-G-A-G-C-T-A-G-G-T-G-C-A-T-C-T-C-G-G-T-C-G-G-T-T-C-G-A-A-C-A-C-G-A-A-A-A-G-G-A-A-T-C-A-A-T-A-3', or being substantially homologous therewith.

18. A recombinant plasmid according to Claim 13 wherein said RDS is obtained from <u>Rhizobium</u> <u>trifolii</u>, the RDS thereof comprising the sequence 5'-G-A-T-C-G-A-C-C-G-A-G-A-G-G-G-C-G-T-C-A-T-T-G-G-G-C-T-C-G-C-T-C-T-G-C-G-G-C-T-T-C-T-T-C-A-G-G-G-G-C-A-C-A-T-G-A-C-A-T-A-T-G-T-G-C-G-A-C-A-T-T-G-T-C-G-T-C-G-C-T-T-T-G-T-C-G-G-C-T-T-C-G-T-G-A-C-A-C-G-G-G-T-T-T-A-G-G-T-T-C-T-T-C-G-G-T-C-C-A-C-T-A-T-T-T-T-A-A-A-C-C-T-C-T-A-A-G-T-G-T-C-T-G-T-G-G-C-A-A-

C-A-A-T-C-T-T-T-T-G-T-T-C-T-G-C-C-T-A-T-C-A-A-T-C-A-C-T-C-A-A-T-T-T-A-C-A-C-G-C-T-T-G-A-A-A-A-T-T-T-T-T-C-A-C-G-G-G-C-T-T-C-G-A-C-G-G-A-C-C-C-T-T-T-T-A-T-G-A-A-A-C-G-G-A-G-A-T-C-G-C-A-T-G-T-C-C-A-T-C-C-G-A-A-A-A-G-G-A-A-G-T-T-C-T-T-3', or being substantially homologous therewith.

19. A recombinant plasmid according to Claim 13 wherein said RDS is obtained from <u>Rhizobium trifolii</u>, the RDS thereof comprising the sequence 5'-A-T-T-G-C-T-G-C-T-C-G-A-T-T-G-C-G-C-T-T-C-T-A-C-T-C-C-G-C-G-G-C-C-T-C-T-T-C-A-G-G-A-G-C-G-A-C-A-G-A-T-G-T-G-A-C-C-A-G-T-T-G-T-C-G-T-C-A-C-C-T-T-T-G-T-C-G-G-C-T-T-C-G-T-G-A-C-A-C-G-C-T-T-T-A-G-G-A-T-T-C-T-T-C-G-G-T-C-C-G-G-T-A-T-T-T-T-A-T-C-C-C-T-C-T-A-A-G-T-G-T-C-T-G-C-G-G-C-A-G-C-A-C-C-A-A-C-T-T-C-C-G-T-T-C-T-G-C-C-C-C-T-T-C-A-A-T-C-A-G-C-T-C-A-A-T-T-G-G-C-A-C-G-A-C-G-C-T-T-G-A-A-A-A-T-T-G-T-T-C-T-C-G-G-G-C-T-G-C-G-A-C-G-G-A-A-C-C-A-C-G-C-G-T-C-C-G-A-T-G-T-C-G-C-G-G-C-A-T-C-C-C-C-T-C-G-G-T-C-G-A-T-T-C-G-A-A-C-A-C-G-A-A-A-A-G-G-A-A-G-A-A-A-T-A-3', or being substantially homologous therewith.

20. A recombinant plasmid according to Claim 13 wherein said RDS is obtained from <u>Rhizobium trifolii</u>, the RDS thereof comprising the sequence 5'-A-T-C-G-A-T-G-C-G-C-G-G-C-T-C-A-T-T-C-G-A-C-C-G-A-G-A-G-T-T-G-T-A-G-C-G-C-A-T-T-G-A-A-G-C-C-G-A-C-C-C-A-C-T-G-C-T-T-T-C-T-C-T-T-C-A-G-G-A-G-C-A-A-C-A-T-G-A-C-A-T-G-T-G-T-C-C-G-A-C-A-T-T-G-T-C-G-T-T-T-C-C-T-T-T-G-T-C-G-A-C-T-T-C-G-A-G-A-C-A-C-G-T-C-T-T-A-G-G-A-T-T-C-T-T-C-G-G-T-C-C-G-A-T-A-T-T-T-T-A-T-C-C-C-T-C-T-A-A-G-T-G-T-T-T-G-C-G-G-C-A-C-A-C-C-A-A-A-T-T-C-C-G-T-T-C-T-G-C-C-A-C-A-T-C-A-A-T-C-C-G-C-C-C-A-G-T-C-T-G-G-C-A-C-G-A-C-G-C-T-T-G-A-A-A-A-T-T-G-T-T-C-T-C-G-G-G-C-T-G-C-G-A-C-G-G-A-A-C-C-A-G-C-C-G-C-G-T-C-C-G-A-T-G-T-C-G-C-G-G-C-A-T-C-A-C-C-T-C-G-G-T-C-G-G-T-T-C-G-A-A-C-A-C-G-A-A-A-A-G-G-A-A-G-C-A-A-T-A-3', or being substantially homologous therewith.

21. A recombinant plasmid according to Claim 13 wherein said RDS is obtained from <u>Rhizobium trifolii</u>, the RDS thereof comprising the sequence 5'-G-A-T-C-T-G-C-T-T-G-C-G-C-G-C-C-T-C-T-T-C-G-G-A-C-T-G-C-A-G-A-A-A-G-T-T-A-A-C-A-A-A-A-C-A-T-G-A-G-A-C-T-G-A-A-G-A-T-G-C-T-C-A-A-G-G-G-A-T-A-A-T-C-G-C-G-C-G-C-T-T-C-G-A-A-A-A-T-G-T-T-T-G-C-G-A-C-A-G-G-C-C-C-C-T-C-T-G-C-T-G-T-C-T-C-T-T-C-A-G-G-A-T-A-C-T-C-A-C-G-A-C-A-T-G-T-G-T-G-C-G-A-C-A-T-T-G-T-C-G-T-C-A-A-C-T-T-T-G-T-C-G-A-C-T-T-C-G-T-G-A-C-A-C-G-T-C-T-T-A-G-G-A-T-T-C-T-T-C-G-G-T-C-C-G-G-T-A-T-T-T-T-A-T-C-C-C-T-C-T-A-A-G-T-G-T-T-T-G-C-G-G-C-A-G-C-G-C-C-A-A-A-T-T-C-C-G-T-T-C-T-G-C-C-A-C-A-T-C-A-A-T-C-C-G-C-C-C-A-G-T-C-

T-G-G-C-A-C-G-A-C-G-C-T-T-G-A-A-A-A-T-T-G-T-T-C-T-C-G-G-G-C-C-G-C-G-A-C-G-G-A-A-C-C-A-C-G-C-G-G-C-C-G-A-G-T-C-G-C-G-G-C-A-T-C-A-C-C-T-C-G-G-T-C-G-G-T-T-C-G-À-A-C-A-C-G-A-A-A-A-G-G-A-A-G-C-A-A-T-A-3', or being substantially homologous therewith.

22. A recombinant plasmid according to Claim 13 wherein said RDS is obtained from Rhizobium trifolii, the RDS thereof comprising the sequence 5'-C-C-G-G-T-A-A-A-C-C-A-T-A-G-C-C-A-T-C-C-A-G-T-C-T-C-G-C-T-A-G-A-G-A-A-C-A-G-C-G-C-C-G-C-T-T-T-T-C-A-A-G-G-C-C-A-G-C-A-T-T-T-G-C-A-T-C-A-A-G-T-G-C-G-C-T-C-G-A-T-C-G-A-C-C-G-A-G-A-G-G-T-G-C-G-T-C-A-T-T-G-G-G-G-C-T-C-G-C-C-C-T-C-T-G-C-G-G-C-T-T-C-T-T-C-A-G-G-G-G-C-A-C-C-A-T-G-A-C-A-T-A-T-G-T-G-C-G-A-C-A-T-T-G-T-C-G-T- C-G-C-T-T-T-G-T-C-G-G-C-T-T-C-G-T-G-A-C-A-C-G-G-T-T-T-A-G-G-A-T-T-C-T-T-C-G-G-T-C-C-A-G-T-A-T-T-T-T-A-A-A-C-C-T-C-T-A-A-G-T-G-T-C-T-G-T-G-G-C-A-G-C-A-C-C-A-A-C-T-T-T-C-G-T-T-C-T-G-C-C-T-A-T-C-A-A-T-C-A-G-C-T-C-A-A-T-T-G-G-C-A-C-G-A-C-G-C-T-T-G-A-A-A-A-T-T-G-T-T-C-A-C-G-G-G-C-T-G-C-G-A-C-G-G-A-G-C-C-G-G-C-T-T-G-T-A-T-G-A-A-A-C-G-G-A-G-A-T-C-A-G-C-A-T-G-T-C-C-A-T-C-C-G-A-A-A-A-A-G-A-A-A-G-T-T-C-T-T-C-G-T-C-A-A-G-C-G-G-G-G-A-C-A-A-G-G-C-A-T-C-G-T-G-T-T-T-G-G-C-A-G-C-T-T-C-A-A-C-G-G-A-A-C-T-A-A-A-A-C-T-G-C-G-G-A-G-A-C-A-A-A-C-3', or being substantially homologous therewith.

23. A process for identifying the species of an unknown Rhizobium isolate comprising the steps of:

(a) purifying DNA of the isolate,

(b) cleaving the DNA of the isolate with a restriction endonuclease to generate isolate DNA restriction fragments of various sizes,

(c) fractionating the isolated DNA restriction fragments on the basis of molecular size,

(d) incubating the isolate DNA restriction fragment fractions with a labelled DNA probe comprising RDS of a known Rhizobium species, under hybridization conditions where substantially homologous DNA segments of at least 100 nucleotides length will hybridize to one another, and

(e) identifying the number of isolate DNA restriction fragments that hybridize with the RDS probe, whereby the species of the Rhizobium isolate coincides with the Rhizobium species whose RDS probe hybridizes with more than one isolate DNA restriction fragment.

24. The process of Claim 23 wherein the RDS probe is derived from an RDS of Rhizobium trifolii, Rhizobium meliloti, Rhizobium leguminosarum or Rhizobium phaseoli.

25. The process of Claim 23 wherein the RDS probe comprises RS-1(a), RS-1(b) RS-2 or RS-3 of Rhizobium trifolii.

26. The process of Claim 23 wherein the RDS probe is derived from pRt607 or pRt642.

Nod−
ANU
851
Tn5
Nod+
ANU882
Kmr
nif
H D
RS 1
RS 3
Nod−
Fix±
pRt587 (pRt032) NODULATION
pRt572 (pRt033)
pRt585
pRt270
pRt514
pRt602
pRt2 68
pRt608
pRt011
pRt578
pRt564
pRt580
0 2kb

FIG. I

1
2
3
4
5
6
7
8
9
10
11
23.1-
9.4-
6.6-
4.4-
2.3 -
2.0 -

FIG. 2

Ava I
Cla I
Cla I
RS I
nif H
Cla I
Ava I
pBR328
Cla I
Ava I
+
DNA ligase
Cla I
nifH
Ava I
RS I
pRt607
Ava I
Cla I
Cla I
Ava I
RS I
nif H
pRt608
Ava I
Cla I
Ava I, cut and religate
Ava I
RSI
pRt607-1
Cla I


FIG. 3

PH
pRt610
H g E P PS E ES
1kb
Sa P S A F T E
RtRS2
100bp


FIG. 4a

FIG. 4b

pRt578

H B Bg Bg C B S H

1kb

Hp Sa Hp Sa Hp Sa Bg

RtRS3

100bp

FIG. 5

pRt564
E
C A Bg C C
E
H
D
K
pRt680
1 kb
pRt608
C
S
A
Sa
Sa Bg
Sa S
nifHDK mRNA
nifH CODING REGION
pRt642
100bp
pRt669
RtRS1